# EUROPEAN PATENT APPLICATION

(11) **EP 2 353 550 A1**
(43) Date of publication of application: **10.08.2011**
(21) Application number: 10001177.4
(22) Date of filing: 05.02.2010
(51) Int. Cl.: A61F 2/78, A61F 2/80, A61F 5/01

(54) **A member for an artificial limb**

(71) Applicant: Nakamura Brace Co.,Ltd., Oda-shi Shimane 694-0305 (JP)
(72) Inventor: Nakamura, Toshiro, Shimane 694-0305 (JP)
(74) Representative: Schildberg, Peter

(57) **Abstract**

A member (1) for an artificial limb (4) comprises a base member (3) which is made of a fiber cloth and has air permeability and a plurality of protuberances (2) which are made of silicon rubber on both of the inside and outside surfaces of the base member, adjoining protuberances being apart to one another. The base member may be tubular or flat.

## Description

### BACKGROUND OF THE INVENTION

### Field of the invention

The present invention relates to a structure of an artificial limb, typified by a prosthetic leg, particularly, a structure of a member to be mounted to a stump of limb of an amputee (or the like people using the artificial limb).

The artificial limb is a device for supplementing function and appearance of a part of human limbs when lost.

Explanation will be given exemplifying a typical prosthetic leg.
The amputee first puts on a member in a shape of socks onto the stump and inserts the stump with the member into a socket of a prosthetic leg's body, completing putting on of the prosthetic leg. The member in the shape of socks was initially provided for preventing the stump from being r ubbed inside the prosthetic leg. And cushioning efficiency and a capability o f suspending the prosthetic leg were then required of the member. The member has evolved into a liner.

### Prior Art

In view of history of improvement of the prosthetic leg, the basis for t he prosthetic leg now usable was built in the late 1940's. Before the time, the prosthetic leg was made from wood (Magnolia obovata, etc),or metal (mainly aluminum). The stump is covered with a bag like socks and inserte d into the socket, and the socket is suspended by use of a waist belt and a shoulder belt. This is called also the insertion type prosth etic leg and quite a few people still regularly use it at present.

Then appeared the so-called "adsorption type prosthetic leg"
wherein a material for the socket employs plastic light in weight, there is used a liner which tightly fit (having no gaps) not only to the socket but also to the stump, and air stagnating in the socket is discharged through a valve, thereby ensuring a capability of suspending. By this, transmission efficiency of force was improved and the suspending belt was made unnecessary, thereby providing a
prosthetic leg which is smart and enables an easy walking. On the other hand, from adopting the liner having the structure of tightly fitting to the socket and stump without gaps, the stump was brought into such poor situation that a member which does not absorb sweat and tightly fits to skin is inserted into plastic socket having no air permeability.

In the 1990's, such prosthetic leg was proposed and very rapidly spread that the liner further progressed to have a pin (a catch pin) at the b ottom, and the socket formed in the prosthetic leg is provided with a hole into which the pin is detachably accommodated. The pin and t he socket are connected without "play" so that "unnecessary piston reciprocation upon walking" quite likely generated in the conventional prosthetic legs is almost completely eliminated. Besides, the pin has such structure that the pin is provided with a lock mechanism so that the pin is automatically locked when it is accommodated into the socket, and the locking can be released readily from the outside.

In detail, correlation between the prosthetic leg's socket and the liner i s almost ideal in the case of this pin type prosthetic leg. Completeness can be achieved if the stump is able to be well accommodated into the liner without troubles and there are found no troubles in use of the prosthetic leg.

However, it is hard to mount safely and surely a metal pin (usually made of stainless steel) having a considerable length (usually about 7cm) to the liner mainly made of silicon rubber. And engineers to manufacture prosthetic limbs cannot carry out the above work with hand-operation. Hence, there has been adopted such way that any liners h aving sizes close to relevant sizes are selected from ready-made liners in various sizes and finely adjusted to obtain a liner suitable for a concerned amputee. The liner obtained from this way is quite expensive.

For the makers, setting of various sizes of the liner leads to rise of unit price. Only rough sizes are prepared actually. As a result, there is naturally a limit to arrange, by adjustment or re-machining, any liners of specific sizes not just relevant to the amputee. And the amputee while anxious about existence of the metal pin n ear the stump is forced to use the prosthetic limbs which even not fit in siz e to the amputee and even quite expensive.

For the amputee using the prosthetic leg, an ideal prosthetic leg is one that feels return of a healthy leg. The adapting part between the stump and the liner is farthest from the ideal state at present. If there are much gaps between the stump and the liner, the stump is unstable inside the liner, and it is hard for the amputee to make walking motion. In case that the gaps between the stump and the liner are eliminated to cause the stump and the liner to tightly fit to each other, there is no air permeability, causing a feel of being sticky with sweat, generating stink, getting sweaty and thereby making troubles of the skin. Moreover, the liner is easily taken off of the stump without the tight fitting, while the tight fitting makes hard to remove the liner from the stump.

The liner used for the pin type prosthetic leg is so structed that silico n rubber which adjusted of hardness to cause a feel of fitting to the skin is arranged in an inner layer (the surface to contact with the stump) and the liner is wrapped with a fibers bag having stretching efficiency. The liner is mounted or fit to the stump in such manner that the liner is fit and fixed onto the stump as pressing the whole surface of the stump. But, this structure has actually not have room for "fine adjustment". As a result, this is merely the case that only such an amputee whose stump occasionally fortunately adapts in size to the foregoing rough sizes of ready-made liners obtains a favourable prosthetic leg.

### SUMMARY OF THE INVENTION

The inventor zealously studied in consideration of the above problems in order to develop a liner which is multipurpose and provided at a low cost and achieved the present invention which is characterized in that a member for an artificial limb comprising: a base member which is made of a fiber cloth and has air permeability; and a plurality of protuberances which are made of silicon rubber on both of the inside and outside surfaces of the base member, adjoining protuberances being apart to one another.

### BRIEF DESCRIPTION OF THE DRAWINGS

Fig. 1 is a schematic front view showing an example of a member for artificial limbs according to the present invention.
Figs. 2(a) through 2(d) each show grain-like protuberances on the member for artificial limbs according to the present invention, Fig. 2(a) being a plan view, and Figs. 2(b) through 2(d) schematic perspective views.
Fig. 3 is a schematic perspective view showing an example in the situation that a liner for artificial limbs according to the present invention is about to be used.
Figs. 4(a), 4(b), and 4(c) are each schematic plan views showing other examples of the member for artificial limbs according to the present invention.

### DETAILED DESCRIPTION OF THE INVENTION

In the history of improvement of the prosthetic legs from the insertion type prosthetic leg suspended by a belt, to the type of holding the socket with adsorption force, and further to the catch-pin type prosthetic legs, function that the liner is required to have becomes gradually larger in extent, so that the stump has undergone the se vere situation. The inventor has judged that the cause is that there was n o escaping from the conventional common sense that adaptation between the stump and the inner surface of the liner is achieved only with mutual tight contacting of the materials. This is the starting point of the present invention.

In the present invention, both of the inside and the outside surfaces of the base member are not flat and are provided with a plurality of protuberances. Since the protuberances are made of silicon rubber, although the protuberance themselves do not allow air to permeate them, the adjoining protuberances are each apart from one another to thereby be independent individually, so that the base member ma de of fibers cloth having air permeability is ensured to have air permeability.

Moreover, since the adjoining protuberances are each apart from one another to thereby be independent individually, each protuberance does readily transform when the base member is put on to the stump or is mounted to the socket, so that the base member is adapted to be put on to the stump or mounted to the socket with the protuberances being so transformed. In the motion of walking, each of the protuberances recovers from the transforming and further transforms again to thereby follow change of shape of the base member, resulting in that a suspending force equivalent to the adsorption type is obtained.

The said many protuberances are made of silicon rubber as foregoing. Shape of each protuberance is not particularly defined but may employ various forms such as being semi-spherical, disc-like, conical, or the like. Providing the protuberances on the surfaces of the base member may be carried out with a method of adhering grain-like matters. Otherwise, an appropriate method for this found in the inventor's trial is that a melt having high viscosity is applied (drop-wise) on the surface of the base member little by little and is set with the base member having being impregnated with a part of the applied melt. Size of the protuberances may preferably be 2mm x 2mm through 10mm x 10mm in extent, and about 2 5mm in height. Also, adjoining protuberances are each apart from one another. Regarding provision of the protuberances, proportion of provision of the protuberances on the base member is preferably 30% to 70% in the whole area. Since the peripheral edge part of the base member according to the present invention does not enter into the socket, it does not need to be provided with the protuberances.

The base member is made of a fiber cloth having air permeability but is not particularly defined of the quality of material. Further, the base member may be tubular or flat in shape. In case of using the tubular shape, the base member may be used in the same manner as that of the conventional liner.

In case of forming the base member in a flat shape, first, the stump is wrapped with the flat base member and is then pushed into the socket. A suitable fixing force is decided by adjusting an amount and positional arrangement of the base member so wrapping the stump. The adjusting may be freely repeated. Thus, the work for the flat type base member is not troublesome although needing to get the knack a little to operate.

### Embodiments

Next, further detailed explanation of the invention will be given with referring to the attached drawings.

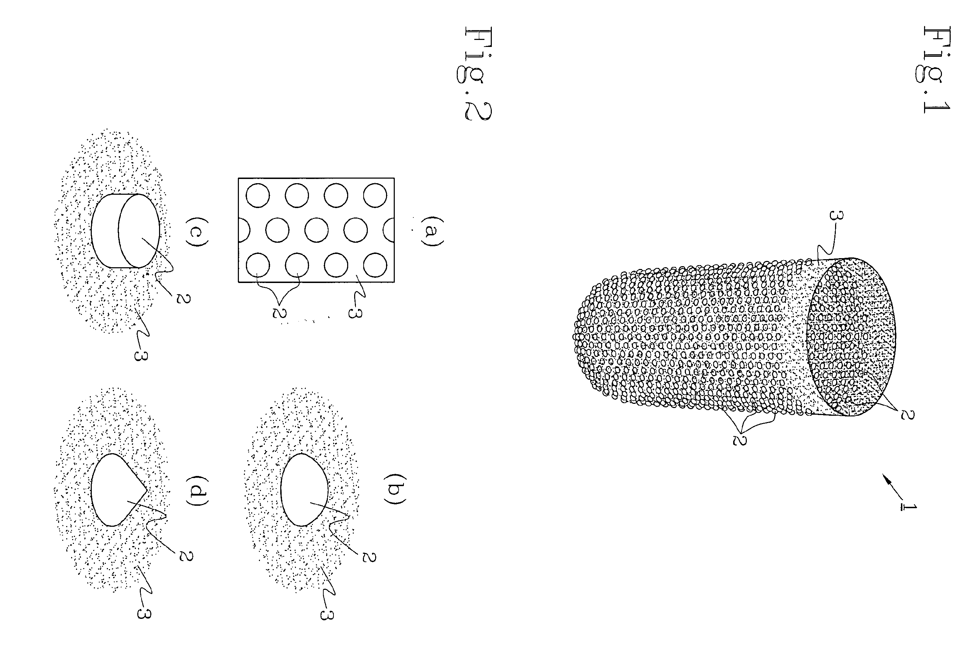

Fig. 1 shows an example of a member 1 for artificial limbs according to the present invention (called hereunder "the member 1 (in accordance with the present invention)").

As clearly seen in the drawing, the member 1 in this example is a tubular matter bottomed, slightly tapered to the bottom and provided on the outside and the inside surfaces with many grain-like
protuberances 2. A base member 3 forming the tubular matter is a fibers cloth having a slight stretchability, on which melt silicon rubber having high viscosity is applied drop-wise to be connected with the base member 3, thereby forming the member 1 in accordance with the present invention. Hence, air permeability of the base member 3 is able to be held even after the grain-shaped protuberances 2 are formed on the surfaces of the base member 3.

Fig. 2 shows the grain-like protuberances 2. The grain-like protuberances 2 are provided in a large number, on the base member 3 as shown in Fig. 2(a), with adjoining protuberances 2 being apart to one another. Shapes of the protuberances may employ variously semi-spherical shown in Fig. 2(b), column-like in Fig. 2(c), conical in Fig. 2(d), or the like.

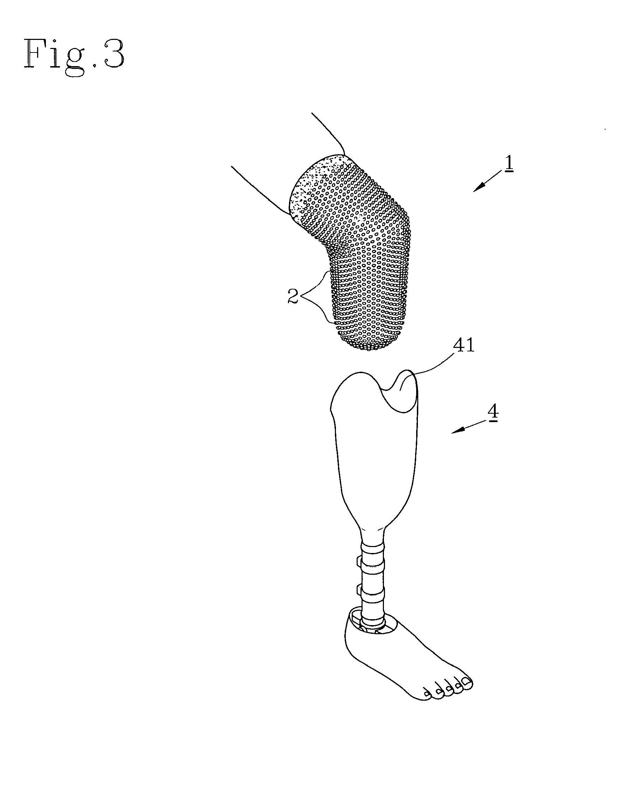

The method of using the member 1 according to the present invention starts with mounting the member 1 onto the stump of the amputee as shown in Fig. 3. In this situation, the grain-like protuberances 2 on the inner surface side of the member 1 are in contact with the surface of the stump. Although the member 1 is not that it tightly fits (as having no gaps) not only to the socket but also to the stump, it is essentially necessary to have specific sizes enough to prevent the member 1 from readily falling from the stump in this situation.

Then, the stump is fit into a socket 41 of a prosthetic leg 4. The fitting work is somewhat troublesome. Air compressed as the stump is being fit into the socket 41 leaks from the base member 1 through spaces defined by steps formed with the grain-like protuberances 2, or thanks to air permeability of the base member 3 itself. Hence, the fitting work is quite readily carried out in comparison with the case using the conventional adsorption type socket (wherein air is discharged through a valve provided at the bottom of the socket).

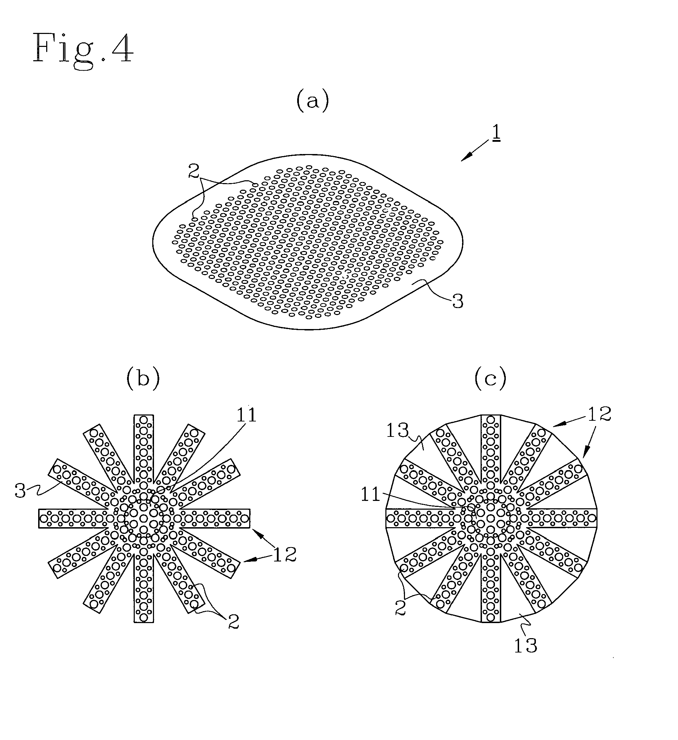

Figs. 4(a), 4(b) and 4(c) show other examples of the present invention. The member 1 in accordance with the present invention in this example is in a flat shape but not tubular. The member 1 shown in Fi g. 4(a) is almost in a rectangular shape, that in Fig. 4(b) in shape having a central disc-like part 11 with belt parts 12 extending radially of the disc-like part 11, and that in Fig. 4(c) in shape connecting the belt parts 12 by means of a thin film 13. In these features, the grain-like protuberances 2 are provided on both of the front and rear sides. To be noted is that there is no existence of grain-like protuberances 2 on the thin film 13 sections shown in Fig. 4(c). In use of the member 1, first, the stump is applied, at its utmost end, to around the central part of the member 1 to be wrapped entirely and then fit into the socket 41. The central part is to be subjected to load and may therefore be made thicker than other parts around the central part. Since the member 1 is flat, a part of the member 1 when applied farther away from the stump is folded more largely. This is to be considered upon putting on the member 1 to the stump, so that experience is needed. Meanwhile, the member 1 itself, which is not provided with any members such as a metal pin or the like, d oes not need to be machined or adjusted, thereby enabling the member 1 to be provided at a quite low cost.

The foregoing explanation on the present invention has been given exemplifying a prosthetic leg. The present invention is properly applicable to a prosthetic arm (not shown).

### Effects of the invention

The member for an artificial limb according to the present invention is quite highly advanced and has the following effects.
(1) Air permeability of the member solves or mitigates the problems of the amputee's stump getting sweaty from a sweat, the member's easily falling due to sweat serving as lubrication, and the cause of troubles on the skin.
(2) The grain-like protuberances are provided independently on the member, so that they readily transform when pressed. By this, there is rea lized comfortable using the artificial limbs without precise arranging sizes of the member.
(3) There is no need of any members such as a metal pin or the like which causes a sense of anxiety in use of the artificial limbs.
(4) The member is provided at a low cost.

## Claims

1. A member for an artificial limb comprising: a base member which is m ade of a fiber cloth and has air permeability; and a plurality of protuberances which are made of silicon rubber on both of the inside and outside surfaces of the base member, adjoining protuberances being apart to one another.

2. A member for an artificial limb as set forth in claim 1 wherein the base member is flat.
